# EUROPÄISCHE PATENTANMELDUNG

(11) **EP 1 216 649 A2**
(43) Veröffentlichungstag der Anmeldung: **26.06.2002**
(21) Anmeldenummer: 01129832.0
(22) Anmeldetag: 14.12.2001
(51) Int. Cl.: A61B 1/24

(54) **Handinstrument zur medizinischen, insbesondere zahnmedizinischen Diagnose**

(30) Priorität: 15.12.2000 DE 10062737
(71) Anmelder: Sirona Dental Systems GmbH, 64625 Bensheim (DE)
(72) Erfinder: Landgraf, Hermann, 64653 Lorsch (DE)
(74) Vertreter: Sommer, Peter

(57) **Zusammenfassung**

Es wird ein Handinstrument zur medizinischen, insbesondere zahnmedizinischen Diagnose vorgestellt welches ein Instrumentengehäuse (5) aufweist, an dessen proximalem Ende eine vorzugsweise auswechselbar gehalterte Untersuchungssonde (3) und an dessen distalem Ende eine Bildwiedergabeeinrichtung (4) mit Bildschirm (9) angeordnet sind und welches im Inneren mindestens eine Beleuchtungseinrichtung (23) mit Spannungsquelle (22), Lichtübertragungsmittel sowie Mittel zur Bilderzeugung enthält. Die Bildwiedergabeeinrichtung (4) ist zumindest um eine senkrecht zur Längssymmetrieachse (7) des Handinstruments (2)verlaufende Vertikalachse (8) schwenkbar am Instrumentengehäuse (5) angeordnet.

## Beschreibung

Die Erfindung betrifft ein Handinstrument zur medizinischen, insbesondere zahnmedizinischen Diagnose, entsprechend dem Oberbegriff des Patentanspruches 1.

Handinstrumente der vorgenannten Gattung werden zu vielfältigen Untersuchungen in Körperhöhlen eines Menschen eingesetzt. Es besteht dabei der Wunsch, das zu untersuchende Objekt nicht nur dem Behandler, sondern auch einer ihm assistierenden Person und/oder dem Patienten selbst am Bildschirm zeigen zu können.

Die bisher gebräuchlichen, als Handinstrument ausgebildeten Untersuchungssonden enthalten im wesentlichen nur eine zur Bilderfassung notwendige Optik mit Elektronik, in der Regel eine miniaturisierte Videokamera. Die Bildwiedergabe erfolgt über einen extern des Behandlers aufgestellten Bildschirm. Obgleich solche Untersuchungssonden sehr klein und damit handlich ausgebildet sein können, ist die Präsentation der Bildwiedergabe über den extern Bildschirm insofern nicht zufriedenstellend, als der Behandler, wenn er sich das Objekt am Monitor ansehen will den Blickkontakt vom Objekt abwenden muß.

Zur Beseitigung dieses Nachteils ist es aus der US-PS 5 879 289 bekannt, die Bildwiedergabeeinrichtung direkt am Handinstrument anzuordnen. Bei dem bekannten Handinstrument sind alle notwendigen Komponenten wie Lichtquelle mit Fiberoptik zur Ausleuchtung des Untersuchungsfeldes, Mittel zur Bilderzeugung (CCD), sowie Mittel zur Bildübertragung an die Bildwiedergabeeinrichtung im Handinstrument enthalten. Die Bildwiedergabeeinrichtung besteht aus einem auf dem Gehäuse des Handinstruments aufgesetzten Miniaturmonitor. Gemäß einer der in der Patentschrift beschriebenen Varianten können die im Bilderzeuger gewonnenen Signale auch an einen extern angeordneten Monitor übertragen werden. Dadurch soll erreicht werden, dass ein und dasselbe Handinstrument in verschiedenen Untersuchungsräumen eingesetzt werden kann, wobei die Signale über ein an das Handinstrument anschließbares Übertragungskabel an eine zentrale Auswerteelektronik mit Monitor gesandt werden. Die Spannungsversorgung erfolgt wahlweise über eine integriert oder extern des Handinstruments angeordnete Spannungsquelle.

Der Miniaturmonitor ist mittels eines Scharniers mit quer zur Längssymmetrieachse des Handinstruments verlaufender Horizontalachse klappbar am Gehäuse angeordnet und kann so aus einer nach unten geklappten Nichtgebrauchsstellung in eine Gebrauchsstellung hochgeklappt werden.

Nachteilig bei dem bekannten Handinstrument ist, dass die Position des Monitors im hochgeklappten Zustand nicht weiter verändert werden kann. Die Blickrichtung auf den Monitor ist damit im wesentlichen durch die Längssymmetrieachse des Handinstruments vorgegeben. Eine solche 'starre' Anordnung ist insbesondere dann von Nachteil, wenn eine Kommunikation mit anderen Personen die nicht den direkten Blick auf das Objekt haben, erwünscht ist. Der Patient beispielsweise kann das Bild überhaupt nicht betrachten wenn die Sonde auf das Objekt gerichtet ist. Ein weiterer Nachteil ist darin zu sehen, dass das bekannte Handinstrument keine ergonomisch gute Handhabung bietet.

Der im Patentanspruch 1 angegebenen Erfindung liegt die Aufgabe zugrunde, demgegenüber eine Verbesserung zu schaffen und ein Handinstrument der eingangs genannten Gattung anzugeben, welches ergonomisch sowohl für Rechtshänder als auch für Linkshänder gleich gut zu handhaben ist und welches die Möglichkeit bietet, auch einem neben dem Benutzer des Handinstruments befindlichen Betrachter, unter Beibehaltung der Ausrichtung der Sonde auf das zu betrachtende Objekt, einen Blick auf den Monitor geben zu können.

Dadurch, dass gemäß der Erfindung die Bildwiedergabeeinrichtung um eine Vertikalachse schwenkbar ist, kann der Bildschirm von einer Mittenstellung aus nach beiden Seiten geschwenkt und damit unterschiedlichen Anforderungen und Bedürfnissen angepasst werden. Insbesondere kann so das Handinstrument gleichermaßen für Rechtshänder und Linkshänder eingesetzt werden.

Eine optimale Anpassung läßt sich erzielen, wenn, wie gemäß einer vorteilhaften Ausgestaltung der Erfindung vorgeschlagen wird, die Bildwiedergabeeinrichtung allseitig schwenkbar am Gehäuse gehaltert ist. Hierzu kann es vorteilhaft sein, wenn die Bildwiedergabeeinrichtung proximalseitig einen kalottenförmigen Ansatz aufweist, der in einem entsprechend ausgebildeten Endteil des Instrumentengehäuses verstellbar gefasst ist.

Weitere vorteilhafte Ausgestaltungen und Weiterbildungen der Erfindung ergeben sich aus den weiteren Ansprüchen und der nachfolgenden Beschreibung eines Ausführungsbeispieles der Erfindung mit mehreren Varianten. In der Zeichnung zeigen:
Figur 1 eine Ausführung eines Handinstruments nach der Erfindung, in perspektivischer Ansicht,
Figur 2 das distale Ende des Handinstruments in einer gegenüber Figur 1 um 90º gedrehten Ansicht, teilweise im Schnitt,
Figur 3 eine Variante zu der Ausführung nach Figur 2,
Figur 4 eine weitere Variante zu der Ausführung nach Figur 2.

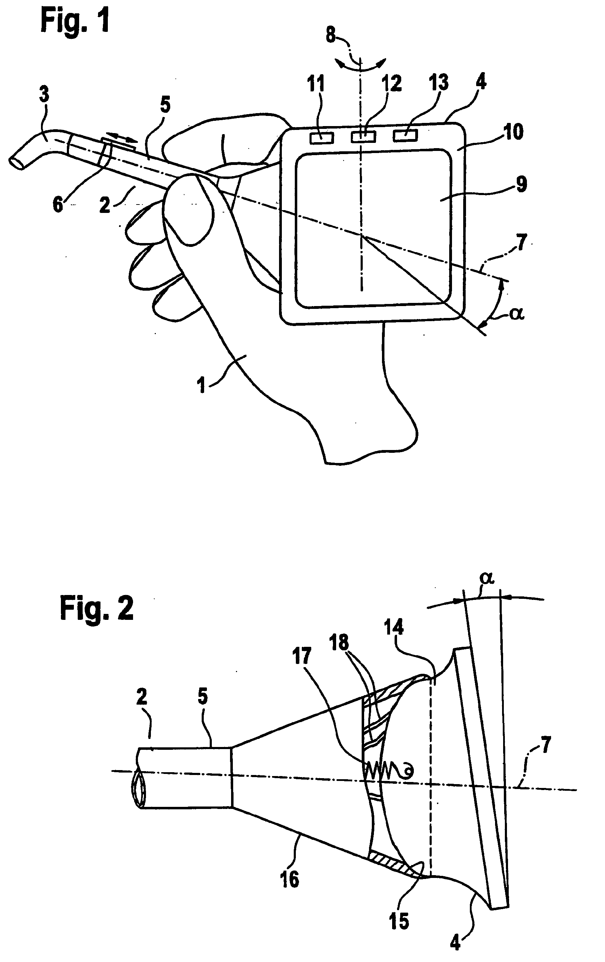

Die Figur 1 zeigt in einer schaubildlichen Darstellung ein in der Hand 1 eines Benutzers gehaltenes Handinstrument 2 welches proximalseitig eine Untersuchungssonde 3 und distalseitig eine Bildwiedergabeeinrichtung 4 aufweist. Die Untersuchungssonde 3 ist vorteilhafterweise auswechselbar am Gehäuse 5 des Handinstruments 2 gehaltert. Mit 6 ist ein in Pfeilrichtung betätigbarer Schieber bezeichnet, mit dem eine im Gehäuse 5 angeordnete Zoomoptik zur Vergrößerung bzw. Verkleinerung des Bildes an der Bildwiedergabeeinrichtung 4 verstellt werden kann.

Die Bildwiedergabeeinrichtung 4 ist in Bezug auf die Längssymmetrieachse 7 des Handinstruments 2 von einer Mittenstellung aus nach beiden Seiten bis zu einem Winkel α von ca. 45º um eine zur Längssymmetrieachse 7 senkrechte Vertikalachse 8 schwenkbar gehaltert. In der dargestellten Ausführung wird das Handinstrument 2 von einem Rechtshänder gehalten. Die Bildwiedergabeeinrichtung 4 ist dabei nach links, also zu diesem Betrachter hin geschwenkt, so dass der Betrachter ein Bild auf den Bildschirm 9 in ergonomisch günstiger Haltung betrachten kann.

Damit eine Bildbetrachtung in gleich günstiger Weise auch von einem Linkshänder möglich ist, ist die Bildwiedergabeeinrichtung 4, wie bereits angesprochen und nachfolgend noch näher erläutert, auch in die entgegengesetzte Richtung um den Winkel α schwenkbar angeordnet.

Besonders vorteilhaft ist eine allseitige Schwenkbarkeit; also nicht nur seitwärts, sondern auch nach unten und oben. Damit läßt sich eine optimale Anpassung an unterschiedliche Blickrichtungen, insbesondere auch für unterschiedlich positionierte Betrachter des Bildschirms 9 erreichen.

Der Bildschirm 9 ist in einem Rahmen 10 gefasst, an dem mehrere Bedienelemente 11, 12, 13 zur Veränderung des Bildstatus wie Bildvergrößerung, Bildverkleinerung und Bildspeicherung, angeordnet sind.

Die Figur 2 zeigt das distale Ende des Handinstruments in einer gegenüber Figur 1 um 90º gedrehten Ansicht, teilweise im Schnitt. Die Bildwiedergabeeinrichtung 4 weist ein kalottenförmiges Gehäuse 14 auf welches in einer entsprechend gestalteten Kontur 15 des konisch ausgeführten Endteils 16 des Handinstruments 2 gefasst ist. Vorteilhafterweise ist die Bildwiedergabeeinrichtung 4 nicht rotationssymmetrisch ausgebildet, sondern im Bereich des Bildschirms mit einer Abschrägung versehen. Die Abschrägung kann beispielsweise den besagten Winkel α einschließen.

Die Bildwiedergabeeinrichtung 4 wird durch beidseitig vorhandene Zugfedern 17 am Endteil 16 so gehalten, dass eine allseitige Schwenkbarkeit in der Kalottenführung gegeben ist. Zusätzlich oder alternativ kann das Endteil 16 am vorderen Teil des Handinstruments drehbar gehaltert sein, so dass es um wenigstens 180º um die Längssymmetrieachse 7 gedreht werden kann. Mit 18 sind diverse in das Gehäuse der Bildwiedergabeeinrichtung 4 führende Leitungen bezeichnet.

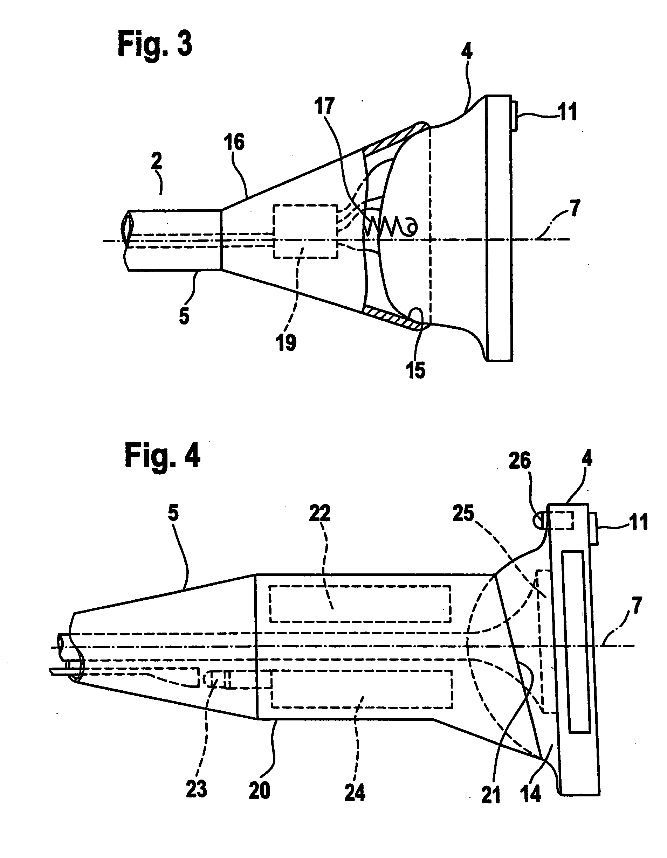

Die Figur 3 zeigt eine Variante, bei der die Bildwiedergabeeinrichtung 4 symmetrisch ausgebildet ist. Auch bei dieser Ausführung ist durch die Kalottenführung eine allseitige Schwenkbarkeit der Bildwiedergabeeinrichtung 4 gegeben. Die zur Bilderzeugung und Bildaufbereitung notwendige Elektronik 19 ist hier im konischen Endteil 16 des Instrumentengehäuses untergebracht.

Bei der in Figur 4 dargestellten Version ist zur besseren Anpassung das im wesentliche zylindrische Endteil 20 mit einer Abschrägung 21 versehen. Die Halterung am Endteil 20 erfolgt wie vorbeschrieben über nicht dargestellte Zugfedern.

Im Inneren des zylindrische Endteils 20 sind eine Versorgungsquelle 22 in Form ein oder mehrerer Batterien oder Akkus, eine Lichtquelle 23, sowie eine Elektronik 24 zur Bilderzeugung und Bildaufbereitung untergebracht. Die Lichtübertragung zur Ausleuchtung des Untersuchungsfeldes und zur Bildübertragung vom Objekt zum Bilderzeuger (CCD) erfolgt in an sich bekannter Weise mittels geeigneter Faseroptik, die in der Figur nicht näher bezeichnet ist.

Damit ein problemloser Wechsel der Batterien oder der Akkus möglich ist, kann das Endteil 20 mit einem entsprechend ausgebildeten Schacht mit Deckel versehen sein. Bei Verwendung von Flachakkus können diese auch im kalottenförmigen Gehäuse 14 untergebracht sein. Die Anordnung solcher Flachakkus ist durch die Position 25 angedeutet. Durch eine solche Maßnahme kann das Gehäuse des Handinstruments sehr viel schlanker gestaltet werden als bei einer Anordnung der Batterien oder Akkus im Endteil 16.

Zur Bildübertragung an eine nicht dargestellte, extern des Handinstruments angeordnete Empfangseinrichtung ist an der Rückseite der Bildwiedergabeeinrichtung 4 ein Infrarotsender 26 angeordnet. Der IR-Sender 26 ist mit der Elektronik 24 verbunden, die vorteilhafterweise auch einen Bildspeicher umfaßt.

## Patentansprüche

1. Handinstrument zur medizinischen, insbesondere zahnmedizinischen Diagnose, mit einem Instrumentengehäuse (5) an dessen proximalem Ende eine vorzugsweise auswechselbar gehalterte Untersuchungssonde (3) und an dessen distalem Ende eine Bildwiedergabeeinrichtung (4) mit Bildschirm (9) angeordnet sind und in dessen Inneren mindestens eine Beleuchtungseinrichtung (23) mit Spannungsquelle (22) und Lichtübertragungsmitteln sowie Mittel zur Bilderzeugung enthalten sind, **dadurch gekennzeichnet, dass** die Bildwiedergabeeinrichtung (4) zumindest um eine senkrecht zur Längssymmetrieachse (7) des Handinstruments (2)verlaufende Vertikalachse (8) schwenkbar am Instrumentengehäuse (5) angeordnet ist.

2. Handinstrument nach Patentanspruch 1, **dadurch gekennzeichnet, dass** die Bildwiedergabeeinrichtung (4) allseitig schwenkbar am Instrumentengehäuse (5) gehaltert ist.

3. Handinstrument nach Patentanspruch 2, **dadurch gekennzeichnet, dass** die Bildwiedergabeeinrichtung (4) proximalseitig einen kalottenförmigen Ansatz (14) aufweist, der in einem entsprechend ausgebildeten Endteil (16, 20) des Instrumentengehäuses (5) verstellbar gefasst ist.

4. Handinstrument nach Patentanspruch 3, **dadurch gekennzeichnet, dass** das Endteil (16, 20) des Instrumentengehäuses (5) kegelförmig ausgebildet ist.

5. Handinstrument nach einem der Patentansprüche 2 bis 4, **dadurch gekennzeichnet, dass** das Endteil (16, 20) des Instrumentengehäuses (5) um die Längssymmetrieachse (7) des Handinstruments (2) drehbar gehaltert ist.

6. Handinstrument nach einem der Patentansprüche 1 bis 5, **dadurch gekennzeichnet, dass** das Endteil (20) des Instrumentengehäuses (5) mit einer Abschrägung (21) versehen ist.

7. Handinstrument nach einem der Ansprüche 1 bis 6, **dadurch gekennzeichnet, dass** im kalottenförmigen Ansatz (14) eine Miniaturversorgungsquelle (25) angeordnet ist.

8. Handinstrument nach einem der Ansprüche 1 bis 7, **dadurch gekennzeichnet, dass** im kalottenförmigen Ansatz (14) Mittel zur Erzeugung eines Videobildes angeordnet sind.

9. Handinstrument nach einem der Ansprüche 1 bis 8, **dadurch gekennzeichnet, dass** die Bildwiedergabeeinrichtung (4) einen Sender (26) zur drahtlosen Datenübertragung der Bildinformationen an eine extern des Handinstruments angeordnete Empfangseinrichtung enthält.

10. Handinstrument nach Anspruch 9, **dadurch gekennzeichnet, dass** der Sender (26) ein IR-Sender ist.

11. Handinstrument nach einem der Ansprüche 1 bis 10, **dadurch gekennzeichnet, dass** der Rahmen (10) des Bildschirmes (9) mit Bedienelementen (11 - 13) zur Veränderung des Bildwiedergabestatus (Bildverkleinerung/-Vergrößerung/Bildspeicherung) versehen ist.
